Europäisches Patentamt

European Patent Office

Office européen des brevets

(19)

(11) Numéro de publication: **0 278 794**
A1

## (12) DEMANDE DE BREVET EUROPEEN

(21) Numéro de dépôt: 88400020.9

(22) Date de dépôt: 06.01.88

(51) Int. Cl.⁴: **C 12 R 1/365**
C 12 P 1/06, C 11 B 3/00,
A 23 C 13/16, A 23 D 3/02,
A 23 D 5/02

(30) Priorité: 06.01.87 FR 8700052

(43) Date de publication de la demande:
17.08.88 Bulletin 88/33

(84) Etats contractants désignés:
AT BE CH DE ES FR GB GR IT LI LU NL SE

(71) Demandeur: MONSERBIO
25 Faubourg des Balmettes
F-74000 Annecy (FR)

(72) Inventeur: Chosson, Patricia
l'Ecluse Chemin Passelis
F-31450 Montgiscard (FR)

Deshayes, Christian
17, rue des Couteliers
F-31000 Toulouse (FR)

Frankinet, Jean
20, allée des Fauvettes
F-56000 Vannes (FR)

(74) Mandataire: Gillard, Marie-Louise et al
Cabinet Beau de Loménie 55, Rue d'Amsterdam
F-75008 Paris (FR)

Le demandeur a présenté une déclaration conformément à la règle 28 (4) CBE (remise d'un échantillon uniquement à un expert).
Numéro d'ordre de(s) dépôts: I-636 (CNCM).

(54) Procédé de biodégradation des stérols.

(57) L'invention concerne un procédé de biodégradation des stérols contenus dans une matière grasse alimentaire qui consiste à mettre à incuber au contact de la matière grasse des bactéries capables de dégrader les stérols.

EP 0 278 794 A1

# 0 278 794

**Description**

## Procédé de biodégradation des stérols.

La présente invention concerne un procédé microbiologique de dégradation des stérols dans les matières grasses alimentaires.

L'invention trouve principalement son application dans la fabrication de matières grasses alimentaires à teneur réduite en stérols.

On sait que,chez les personnes ayant une alimentation riche en cholestérol, on constate une altération de la paroi des artères : l'athérosclérose due en partie à une déposition anormale de cholestérol dans la paroi (J.A.M.A., Vol, 253, 1985, pages 2080 à 2086).

On sait également que le cholestérol est essentiellement apporté dans l'alimentation humaine par les matières grasses d'origine animale. Parmi celles-ci, l'une des plus couramment et réguliérement consommées est le beurre qui contient, pour 100 g, entre 220 et 340 mg de stérols dont 98 % sont représentés par le cholestérol.

La gravité que peut revêtir l'athérosclérose a précisément conduit à rechercher des techniques permettant la préparation de matières grasses alimentaires et notamment de beurre ou de compositions à base de matière grasse laitière dont la teneur en cholestérol est réduite.

Les techniques connues pour la dégradation des stérols mettent en oeuvre des méthodes physico-chimiques.

L'utilisation de solvants a par exemple été envisagée mais les solvants les plus adaptés, tels que l'acétone ou le méthanol sont incompatibles avec un usage alimentaire.

Des méthodes d'adsorption sur colonne, telles que par exemple une chromatographie sur une colonne contenant des triglycérides, ont été également proposées. Mais il s'agit de méthodes coûteuses et dont la mise en oeuvre s'accompagne d'une perte significative d'une partie de la matière grasse soumise au traitement.

On peut encore citer une méthode de microdistillation décrite dans le brevet FR-A-2319300 dont l'utilisation s'est avérée peu adaptée à une production industrielle.

La demanderesse apporte une solution aux inconvénients de l'art antérieur en proposant un procédé de dégradation des stérols mettant en oeuvre des bactéries.

La présente invention concerne précisément selon un premier aspect un procédé microbiologique de dégradation des stérols contenus dans les matières grasses alimentaires.

Ce procédé se caractérisé en ce que l'on met à incuber au contact de la matière grasse des bactéries capables de dégrader au moins l'un des stérols qu'elle contient. Il vise donc selon les cas la dégradation du cholestérol lorsque la matière grasse est d'origine animale et la dégradation des stérols, tels que le sitostérol, le stigmastérol ou l'ergostérol, lorsque la matière grasse est d'origine végétale.

Le procédé selon l'invention consiste essentiellement à mettre des bactéries, apportées de préférence sous forme d'une suspension,éventuellement concentrée, au contact de la matière grasse. Ce contact s'effectue de préférence sous agitation, à l'aide par exemple d'un agitateur rotatif, de préférence à la température la plus favorable pour la souche mise en oeuvre. Cette incubation est en général inférieure à 24 h.

Il est possible, en outre, dès la fin de l'incubation, soit de procéder à la séparation des corps bactériens et de la matière grasse, par exemple par décantation ou par centrifugation, soit d'inactiver les bactéries par exemple à l'aide d'un traitement thermique approprié appliqué à la matière grasse.

Les bactéries, mises au contact de la matière grasse, sont apportées de préférence sous la forme d'une suspension. Cette suspension peut notamment résulter de l'incubation d'un milieu de culture liquide préalablement ensemencé à l'aide desdites bactéries. Selon une autre variante, les bactéries, après séparation des autres constituants du milieu de culture ayant servi à leur obtention, sont mises en suspension dans un liquide approprié à un usage alimentaire, tel que par exemple de l'eau déminéralisée additionnée de chlorure de sodium à raison de 9 g par litre.

Toutes sortes de matières grasses, soit d'origine animale, soit d'origine végétale ou leurs mélanges peuvent être soumises au procédé selon l'invention. Il peut s'agir notamment de matières grasses dérivées du lait, telles que la matière grasse laitière anhydre ou MGLA, le beurre ou la crème laitière ainsi que d'autres matières grasses telles que la margarine, les huiles végétales ou les huiles animales. Il peut encore s'agir de suif ou par exemple de saindoux.

Pour la mise en oeuvre du procédé, on peut utiliser toute souche bactérienne capable d'assurer la biodégradation du cholestérol ou d'un autre stérol d'intérêt.

D'une manière préférée, on utilisera la souche du genre Nocardia dont le déposant a effectué, en date du 3 décembre 1986, le dépôt d'une culture auprès de la Collection Nationale de Cultures de Microorganismes ou CNCM (Paris - France), qui lui a attribué la référence I-636.

L'invention concerne donc selon un autre aspect l'utilisation d'une souche bactérienne présentant les caractéristiques de la souche CNCM I-636

La souche CNCM I-636 a été isolée à l'occasion d'un programme de screening réalisé à partir d'échantillons de sols dans la région de Labège (Toulouse - France).

A partir des échantillons mis en suspension dans de l'eau contenant 9 g par litre de chlorure de sodium, des cultures ont été réalisées sur deux milieux solides d'enrichissement dont les caractéristiques sont données ci-dessous :

Milieu 1

Composition
Glycérol................. 20 g
L-arginine, HCl.......... 2,5 g
NaCl.................... 10 g
CaCO₃.................... 0,1 g
FeSO₄,7H₂O............... 0,1 g
MgSO₄,7H₂O............... 0,1 g
Agar-agar............... 20 g
Cycloheximide........... 50 g
(Actidione®)
pH : 6,5 à 6,8.
Stérilisation : 20 min à 120° C.

Milieu 2
Milieu décrit par SCHAAL D.P. [Zbl. Bakteriol. Parasitenk Infektions Kv. Agg. 220, No 1-3,24 (1972)].

Composition
NaH₂PO₄........................ 1 g
MgS0₄,7H2O.................... 0,5 g
KNO₃ ........................ 2,5 g
Acétate de sodium............. 5 g
Propionate de sodium.......... 5 g
Hydrolysat de caséine sans vitamine......... 10 g
Agar-agar type E.............. 15 g
pH : 7,5
Stérilisation : 20 min à 120°C.
Parmi les souches isolées, la souche CNCM I-636 a été sélectionnée en raison de son aptitude à dégrader les stérols et en particulier le cholestérol.
Afin d'assurer la conservation de la souche CNCM I-636, il est possible de procéder de la manière suivante :
Après 48 h de culture à 28°C sur milieu BHI (Brain Heart Infusion DIFCO® référence 0418), la culture est reprise par 5 ml d'un milieu de conservation pour la congélation dont les caractéristiques sont données ci-dessous, répartie dans des tubes de Nunc, à raison de 1 ml par tube, et conservée à -80°C.

Milieu de conservation

Composition (par litre)
Chlorure de sodium................. 9 g
Chlorure de potassium............. 0,42 g
Chlorure de calcium............... 0,48 g
Bicarbonate de sodium............. 0,2 g
Glycérol......................... 150 g
MOPS (3-(n-morpholino)propanesulfonique acide).... 3 g
pH : 7,0 avant stérilisation.
Stérilisation : 20 min à 120° C.
La souche CNCM I-636 a été caractérisée à l'aide des caractères morphologiques et biochimiques décrits dans les ouvrages et articles suivants :
1. M. Goodgellow, and K.P. Shaal (1979) "Identification methods for Nocardia, Actinomadura, and Rhodococcus" in "Identification Methods for Microbiologists", F.A. Skinner and D.W. Loverlock (ed), Acad. Press., London, p. 261-276.
2. R.M. Kroppenstedt, (1985) "Fatty acid and menaquinone analysis of actinomycetes and related organisms" in "Chemical Methods in Bacterial Systematics", M. Goodfellow and D.E. Minnikin (ed) Acad. Press. London p. 173-179.
3. M. Daffé, M.A. Lanéelle, C. Asselineau, V. Lévy-Frébault et H. David (1983) "Intérêt taxonomique des acides gras des mycobactéries : proposition d'une méthode d'analyse". Ann. Microbiol. (Inst. Pasteur), 134 B, p. 241-256.
La souche CNCM I-636 se caractérise notamment par :
- sa réaction positive à la coloration de Gram.
- son acido-alcoolo-sensibilité mise en évidence par la coloration de Ziehl-Neelsen.
- sa morphologie coloniale : des colonies blanc-crème, pâteuses, à mucoïdes, et légèrement expansives sont obtenues après 18 h de culture, sur le milieu gélosé BHI (Brain Heart Infusion agar, DIFCO® référence 0418).
- sa micromorphologie : on observe sur le milieu BHI la formation de filaments blanc-crème, rampants et dépourvus d'hyphes aériennes sur des cultures de moins de 12 h et la fragmentation des filaments précoce en éléments allongés polymorphes ou coccoïdes. On note l'absence de sporulation.
- ses caractéres culturaux : qui sont présentés dans le tableau no 1 ci-après.

## TABLEAU 1

| Milieu de culture (1) | Croissance (2) | Mycélium primaire | Eléments séparés allongés | Eléments séparés coccoïdes | Pigmentation (3) |
|---|---|---|---|---|---|
| Extrait de levure Extrait de malt (ISP2) | ++ | + | + | - | - |
| Farine d'avoine (ISP3) | + | - | - | + | - |
| Sels inorganiques amidon (ISP4) | + faible | - | - | + | - |
| Glycérol-Asparagine (ISP5) | + | + | + | - | - |
| Heptane-Extrait de levure-fer (ISP6) | ++ | + | + | - | - |
| Tyrosine agar (ISP7) | + faible | + | + | - | - |

* (1) ISP = International Streptomyces Procedure.

* (2) La croissance est évaluée après 20 h d'incubation.

* (3) La pigmentation est observée sur une période de 8 jours.

- La constitution de l'enveloppe cellulaire
Cette enveloppe a les propriétés suivantes :
* Paroi de type IV (présence d'acide mésodiaminopimélique, d'arabinose et de galactose).
* Présence d'acides mycoliques et de phosphatidyléthanolamine.
* Présence d'acides gras :
. en $C_{14}$ saturé (acide myristique)
. en $C_{16}$ saturé (acide palmitique)
. en $C_{16}$ saturé méthylé (acide 10-méthyl-palmitique)
. en $C_{16}$ mono-insaturé (acide palmitoléique)
. en $C_{18}$ saturé (acide stéarique)
. en $C_{18}$ saturé méthylé (acide 10-méthyl-stéarique)
. en $C_{18}$ mono-insaturé (acide oléique).
* Absence d'acides gras de longueur de chaîne supérieure à 18 atomes de carbone.
L'ensemble des caractères ci-dessus présentés indique que la souche CNCM I-636 appartient au genre Nocardia.

D'autres caractères biochimiques présentés dans le tableau 2 ci-après ne permettent pas, considérés dans leur ensemble, par contre de rattacher la souche CNCM I-636 à l'une des espèces de Nocardia possédant des acides mycoliques déjà décrites (M. Goodfellow and D.E. Minnikin 1981 "The genera Nocardia and Rhodococcus" in "The Procaryotes", P. Starr, H. Stolp, H.G. Trüpper, A. Balows and H.G. Schlegel (ed) Springer Verlag, Berlin p. 2016-2027).

TABLEAU 2

| DECOMPOSITION DE | |
|---|:---:|
| L'adénine | + |
| La caséine | − |
| L'élastine | − |
| L'hypoxanthène | − |
| Le testostérone | + |
| La tyrosine | − |
| La xanthine | − |
| RESISTANCE | |
| Au lysozyme | − |
| A la rifampicine | − |
| PRODUCTION | |
| D'uréase | − |
| FORMATION D'ACIDE A PARTIR : | |
| D'adonitol | − |
| De cellobiose | − |
| De méso-érythritol | − |
| De lactose | − |
| De maltose | − |
| De mélèzitose | − |
| D'α-méthyl-D-glycoside | − |

La demanderesse a proposé que la nouvelle espèce représentée par la souche CNCM 1-636 soit nommée Nocardia labegensis.

La souche CNCM 1-636 est remarquable pour son aptitude à biodégrader les stérols contenus dans les matières grasses d'origine animale ou d'origine végétale ainsi que leurs mélanges.

Son utilisation permet d'éliminer, par exemple, le cholestérol libre contenu dans la Matière Grasse Laitière Anhydre (MGLA) en moins de 12 h.

Il a été vérifié, par chromatographie sur couche mince et également en suivant la dégradation du cholesterol à l'aide d'un traceur radioactif, que la souche CNCM I-636 dégrade les stérols en composés de faible poids moléculaires sans formation de produits intermédiaires tels que la Δ4-cholestène-3-one, le 4-androstène-3,17-dione ou le 1,4-androstadiène-3,17-dione.

Dans ce qui suit, des exemples non limitatifs de réalisation de l'invention sont présentés :

Exemples

I - TRAITEMENT DE 4 MATIERES GRASSES ET ANALYSE PAR CHROMATOGRAPHIE SUR COUCHE MINCE, DOSAGE ENZYMATIQUE ET CHROMATOGRAPHIE EN PHASE GAZEUSE.

1- METHODOLOGIE

## 1.1 MATIERES GRASSES TESTEES

Quatre matières grasses ont été testées :
- de la MGLA (France-Beurre ®)
  * prise d'essai : 1,5 g
- de la créme laitière (crème homogénéisée à 60 % de matière grasse ULPAC®)
  * prise d'essai : 1 g
- de l'huile d'arachide (Carrefour®)
  * prise d'essai : 2 g
- une margarine (Astra®)
  * prise d'essai : 1,5 g

La MGLA et la margarine sont portées à environ 50°C pour être utilisées à l'état liquide.

## 1.2 SOUCHE BACTERIENNE MISE EN OEUVRE

La souche CNCM I-636 a été utilisée.

## 1.3 MODE OPERATOIRE

a) Phase de production de biomasse

1 ml de la suspension de la souche CNCM I-636 conservée à -80°C est mis en culture dans 40 ml d'un milieu de culture.

Deux milieux ont été utilisés : les milieux A et B dont les caractéristiques sont indiquées ci-après :

Milieu A :

* Composition par litre :
  . $(NH_4)_2SO_4$............2 g
  . $CaCl_2,2H_2O$............0,01 g
  . $FeSO_4,7H_2O$..............0,01 g
  . $K_2HPO_4$...............2 g
  . $MgSO_4,7H_2O$........... 0,2 g
  . Glycérol............ 10 g
  . Extrait de levure (DIFCO® - référence 0127).... 20 g
* pH : 6,7
* Stérilisation : 20 min à 120°C.

Milieu B

* Composition pat litre :
  . Glucose........................................... 10 g
  . Peptone (DIFCO® - référence 0118)................ 4 g
  . Extrait de viande (DIFCO® - référence 0126)...... 4 g
  . Extrait de levure (DIFCO® - référence 0127)...... 1 g
  . NaCl..............................................2,5 g
* pH : 7
* Stérilisation : 20 min à 120°C.

Après 24 h d'incubation à 28°C sur table rotative (220 rpm), on procède à un repiquage avec un taux d'incubation de 5 % : on prélève 2 ml de la culture obtenue et on les ajoute à 40 ml de milieu neuf du type de celui déjà utilisé. Après une deuxième incubation de 24 h à 28°C sous agitation, on ajoute à la culture obtenue une prise d'essai de la matière grasse à tester à l'état liquide.

b) Phase de bioconversion

La culture ainsi complétée est soumise à une incubation de 12 h à 28°C sous agitation constante.

c) Récupération de la matière grasse traitée

On procède ensuite à une centrifugation (9 000 tours par minute pendant 15 min). Le surnageant, constitué par la matière grasse, est lavé deux fois avec 10 ml d'eau déminéralisée. On récupère ainsi sensiblement toute la matière grasse de la prise d'essai.

## 1.4 ANALYSE DES RESULTATS

On soumet la matière grasse à une analyse qualitative (par chromatographie sur couche mince ou CCM) et à deux dosages (un dosage par chromatographie en phase gazeuse ou CPG et un dosage enzymatique).

### 1. Analyse par CCM

Le milieu réactionnel contenant la matière grasse est acidifié par addition d'HCl (1N) jusqu'à ce que le pH atteigne la valeur de 2,5.

On procède ensuite à une extraction à l'aide de dichlorométhane (deux fois 10 ml). Puis la phase organique est séchée dans une enceinte contenant du sulfate de sodium anhydre et évaporée sous vide.

Le résidu obtenu est soumis à une CCM. Le support chromatographique utilisé est une plaque de verre recouverte d'une mince couche de gel de silice (référence 60 F254-Merck®). L'éluant est un mélange (70/30 v/v) d'hexane et d'acétate d'éthyle. La révélation est réalisée par pulvérisation d'une solution d'acide sulfurique dans de l'éthanol (50/50 v/v) suivie d'un chauffage à 100°C.

Quatre composés témoins sont déposés sur le support : du cholestérol, de l'ergostérol, et deux 17-cétostéroïdes, du 4-androstène-3,17-dione ou AD et du 1,4-androstadiène-3,17-dione ou ADD.

### 2. Dosage enzymatique

Un kit de dosage des stérols totaux, libres ou estérifiés dans les huiles et les graisses, commercialisé par la Société Boehringer, est utilisé. Ce kit met en oeuvre le mode opératoire décrit dans la REVUE FRANCAISE DES CORPS GRAS (Vol. no 4, avril 1986, M. NAUDET et al., p. 167-170).

### 3. Dosage par CPG

Ce dosage est réalisé en suivant le mode opératoire de la Pharmacopée Européenne (Ph. Eur. 2 (I) v. 20.3) en utilisant du stigmastérol comme étalon interne et une solution témoin de cholestérol. Il permet de doser le cholestérol total. La limite de détection est atteinte pour une quantité de cholestérol correspondant à 0,05 % de l'échantillon préparé pour la CPG.

## 2 - ESSAIS

Sept essais ont été réalisés conformément au tableau 3.

TABLEAU 3

| | Matière grasse testée | Milieu de culture de la souche | Verification de l'efficacité | | |
|---|---|---|---|---|---|
| | | | Analyse CCM | Dosage CPG | Dosage enzymatique |
| Essai 1 | MGLA | A | X | | X |
| Essai 2 | MGLA | B | X | X | X |
| Essai 3 | Crème laitière | B | | X | |
| Essai 4 | Margarine | A | X | | X |
| Essai 5 | Margarine | B | X | | |
| Essai 6 | Huile d'arachide | A | X | | X |
| Essai 7 | Huile d'arachide | B | X | | X |

Chacun de ces essais est doublé d'un essai-témoin pour lequel une prise d'essai de la matière grasse à tester à l'état liquide est ajoutée à 40 ml de milieu (A ou B selon le cas) non ensemencé, le mélange obtenu est soumis à une incubation de 12 h à 28°C sous agitation constante puis est traité selon le mode opératoire décrit au paragraphe 1.3 ci-dessus.

## 3 - RESULTATS

### 3.1 ANALYSES PAR CCM

Examen du support après révélation :
On note pour les quatre composés-témoins les résultats suivants :
- cholestérol : Rf = 0,60, couleur rose
- ergostérol : Rf = 0,30, couleur rose
- AD : Rf = 0,30, couleur verte
- ADD : Rf = 0,20, couleur violette.
En ce qui concerne les extraits préparés à partir de la matière grasse traitée, on constate :

1. Pour les essais no 1, 2 et 7 :
L'absence de taches correspondant à des stérols, à l'AD ou à l'ADD.

2. Pour les essais no 4, 5 et 6 :
La présence résiduelle de stérols (les taches sont de moindre dimension et leur coloration est atténuée par rapport à celle des composés témoins).

### 3.2 DOSAGES ENZYMATIQUES
Les résultats sont présentés dans le tableau no 4 :

### TABLEAU 4

|  | Stérols dans le témoin mg/100 g | Stérols après traitement mg/100 g | Pourcentage de disparition des stérols |
|---|---|---|---|
| Essai 1 | 234 | 25 | 89 % |
| Essai 2 | 323,6 | 45,4 | 86 % |
| Essai 4 | 241 | 108 | 55 % |
| Essai 6 | 221 | 64 | 71 % |
| Essai 7 | 161,4 | 59 | 63 % |

On constate que le procédé mis en oeuvre a permis l'élimination, par dégradation, de 55 à 89 % des stérols initialement contenus dans la matière grasse.

### 3.3 DOSAGES PAR CPG
Pour l'un et l'autre des essais 2 et 3, il n'a pas été détecté de cholestérol dans les échantillons préparés à partir de la matière grasse traitée. Compte-tenu de la sensibilité propre à la méthode utilisée, on peut conclure qu'au moins 80 % du cholestérol total initialement contenu dans la matière grasse ont été dégradés par l'action de la souche CNCM I-636.

II - TRAITEMENT DE MGLA ET UTILISATION DU $^{14}$C-CHOLESTEROL MARQUÉ EN POSITION 4 EN TANT QUE TRACEUR RADIOACTIF

### I. MODE OPERATOIRE
Les essais de biotransformation de la matière grasse laitière anhydre (MGLA) sont effectués en fermenteurs de 2 litres.
3 fermenteurs Biolafitte® du type BL.02.1 sont utilisés pour :
- 1 essai-temoin sans ajout de bactéries et,
- 2 essais mettant en oeuvre la souche CNCM I-636.
Chaque réacteur contient 200 ml de MGLA, 250 µCi de 4-$^{14}$C-cholestérol (Amersham®) et 800 ml de milieu de conversion.

### A. Obtention d'une biomasse

\* Préculture :

Le contenu (soit 1 ml) d'une ampoule de la souche conservé à - 70°C, est placé dans une fiole de 250 ml contenant 40 ml de milieu 25 Bis (décrit ci-après). Après 48 h de croissance à 28°C, et sous une agitation de 220 tours par minute, on mesure le pH et la densité optique à 660 nm.

Milieu 25 Bis : composition donnée en g/l d'eau distillée.

$(NH_4)_2SO_4$ ..................... 2
$CaCl_2,7H_2O$...................0,01
$FeSO_4,7H_2O$...................0,01
$K_2HPO_4$ ...................... 2
$MgSO_4,7H_2O$................... 0,2
Glycérol ..................... 10
Extrait de levure ........... 20
pH = 6,7 avant stérilisation (30 minutes à 120°C).

\* Culture :

La souche est repliquée sur du milieu frais à raison de 8 ml de préculture pour 250 ml de milieu 25 Bis contenu dans des fioles de 1 litre soit un taux d'inoculation de 3,2 %.

Après 48 h de croissance à 28°C et sous une agitation de 220 tours par minute, le milieu de fermentation est récupéré, centrifugé à 10450 g pendant 15 minutes. Le culot obtenu est lavé par 250 ml de milieu 540 (décrit ci-après) puis recentrifugé dans les mêmes conditions. Le culot repris par 250 ml de milieu 540 constitue le milieu de conversion.

Celui-ci est reparti dans des fioles à tubulure latérale à raison de 800 ml par fiole, volume nécessaire pour un fermenteur. Milieu 540 : composition donnée en g/l d'eau distillée.

$KH_2PO_4$ ...................... 4
$Na_2HPO_4,12H_2O$......................4
$(NH_4)_2SO_4$........................2
$MgSO_4,7H_2O$................... 0,2
$CaCl_2,2H_2O$......................0,001
$FeSO_4,7H_2O$......................0,001
Extrait de levure .......... 0,5
pH = 7 avant stérilisation (20 minutes à 120°C)

B. Etape de conversion

A 200 ml de MGLA pesés dans des fioles d'un litre à tubulure latérale, sont ajoutés 250 µCi de 4-$^{14}$C-cholestérol solubilisé dans la MGLA en fusion. Après agitation, on ajoute la MGLA dans le réacteur stérile maintenu à 37°C, puis le milieu de conversion. La vitesse d'agitation est de 450 tours par minute et le débit d'air de 0,9 l/minute. Des prélèvements de 25 ml sont effectués, avant mise en incubation (temps 0) puis après, 11 h 30, 22 h 30 et 44 h 30 d'incubation à 37°C, à l'aide de seringues stériles. Ces échantillons, placés dans des tubes en verre préalablement tarés, sont portés à 60°C puis centrifugés à 13220 g pendant 10 minutes. Après centrifugation, les tubes sont à nouveau portés à 60°C : les phases lipidiques et aqueuse sont prélevées et pesées.

C. Piégeage du gaz carbonique

Le piégeage du gaz carbonique produit est réalisé par l'intermediaire de 2 flacons laveurs contenant respectivement 240 et 75 ml d'un melange éthanolamine/eau distillée. Le volume d'éthanolamine est de 40 ml dans le premier piège et de 15 ml dans le second.

Après chaque prélèvement, le piège est vidé, rincé par de l'eau qui est alors pesée.

D. Mesure de la radioactivité

La radioactivité est mesurée par comptage en scintillation liquide dans 10 ml de scintillant Insta-gel® (United Technology Packard). Les prises d'essais sont de 10 mg pour la MGLA, de 50 mg pour la phase aqueuse et de 1 g pour le mélange contenu dans le piège. Les mesures sont effectuées dans un compteur B PACKARD® (TRICARB 4530 - United Technologies).

II. RESULTATS

Les résultats (cf. tableau 5 ci-après) sont exprimés en % de la radioactivité initialement produite. La correction de quenching est effectuée par la méthode du standard externe.

Il est constaté notamment que :
- la quantité de $^{14}$C-cholestérol dans la MGLA est réduite, après traitement, de plus de 96 % ;
- la dégradation du $^{14}$C-cholestérol conduit à la production de gaz carbonique marqué avec un rendement voisin de 50 %.

Ces résultats confirment l'efficacité de la méthode.

**0 278 794**

TABLEAU 5

| | Phase aqueuse | Phase grasse | Phase gazeuse | TOTAL |
|---|---|---|---|---|
| **ESSAI N° 1** | | | | |
| t : 0 | 1,1 | 94,1 | 0,0 | 95,2 |
| t = 11h30 | 41,6 | 3,9 | 50,1 | 95,6 |
| t = 22h30 | 35,4 | 3,7 | 51,6 | 90,7 |
| t = 44h30 | 38,2 | 3,6 | 52,2 | 94,0 |
| | | | | |
| **ESSAI N° 2** | | | | |
| t = 0 | 1,4 | 102,1 | 0,0 | 103,5 |
| t = 11h30 | 30,7 | 3,8 | 52,2 | 86,7 |
| t = 22h30 | 37,4 | 3,7 | 54,0 | 95,1 |
| t = 44h30 | 41,0 | 3,6 | 54,9 | 99,5 |
| | | | | |
| **TEMOIN** | | | | |
| t = 0 | 0,7 | 98,3 | 0,0 | 99,0 |
| t = 11h30 | 0,3 | 97,8 | 0,0 | 98,1 |
| t = 22h30 | 0,3 | 98,0 | 0,0 | 98,3 |
| t = 44h30 | 1,4 | 102,1 | 0,0 | 103,5 |

**Revendications**

1. Procédé de dégradation d'un stérol contenu dans une matière grasse alimentaire, caractérisé en ce que l'on met à incuber au contact de la matière grasse des bactéries capables de dégrader ledit stérol.

2. Procédé selon la revendication 1, caractérisé en ce que les bactéries sont utilisées sous forme d'une suspension.

3. Procédé selon la revendication 2, caractérisé en ce que la suspension de bactéries résulte de l'incubation d'un milieu de culture liquide préalablement ensemencé à l'aide desdites bactéries.

4. Procédé selon la revendication 2, caractérisé en ce que les bactéries sont en suspension dans un liquide approprié à un usage alimentaire.

5. Procédé selon l'une quelconque des revendications 1 à 4, caractérisé en ce que les bactéries appartiennent au genre Nocardia.

6. Procédé selon la revendication 5, caractérisé en ce que les bactéries ont les caractéristiques de la souche déposée auprès de la CNCM sous la référence I-636.

7. Procédé selon l'une quelconque des revendications 1 à 6, caractérisé en ce qu'il comporte en outre une phase de séparation des bactéries et de la matière grasse.

8. Procédé selon l'une quelconque des revendications 1 à 7, caractérisé en ce qu'après incubation les bactéries sont inactivées par un traitement thermique.

9. Souche bactérienne ayant les caractéristiques de la souche CNCM I-636.

10. Matière grasse à teneur réduite en stérols obtenue selon le procédé de l'une quelconque des revendications 1 à 8.

11. Matière grasse laitière anhydre à teneur réduite en cholestérol selon la revendication 10.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | CHEMICAL ABSTRACTS, vol. 97, no. 15, octobre 1982, page 582, résumé no. 126008e, Columbus, Ohio, US; & JP-A-82 86 257 (KANEBO FOODS LTD) 29-05-1982 * Résumé * | 1 | C 12 R 1/365 C 12 P 1/06 C 11 B 3/00 A 23 C 13/16 A 23 D 3/02 A 23 D 5/02 |
| Y | Idem --- | 5 | |
| Y | CHEMICAL ABSTRACTS, vol. 72, 1970, page 111, résumé no. 39959f, Columbus, Ohio, US; K. ARIMA et al.: "Microbial transformation of sterols. I. Decomposition of cholesterol by microorganisms", & AGR. BIOL. CHEM. 1969, 33(11), 1636-43 * Résumé * --- | 5 | |
| A | FR-A-2 413 038 (SOCIETE D'ASSISTANCE TECHNIQUE POUR PRODUITS NESTLE) * Revendication 1 * ----- | 1 | |

DOMAINES TECHNIQUES RECHERCHES (Int. Cl.4)

C 12 R
C 12 P
C 11 B
A 23 C
A 23 L
A 23 D

**Le présent rapport a été établi pour toutes les revendications**

| Lieu de la recherche | Date d'achèvement de la recherche | Examinateur |
|---|---|---|
| LA HAYE | 27-04-1988 | PEETERS J.C. |